# EUROPEAN PATENT APPLICATION

(11) **EP 3 910 647 A1**
(43) Date of publication of application: **17.11.2021**
(21) Application number: 20173910.9
(22) Date of filing: 11.05.2020
(51) Int. Cl.: G16H 40/63, G16H 50/30

(54) **SWEAT SENSOR**

(71) Applicant: Koninklijke Philips N.V., 5656 AG Eindhoven (NL)
(72) Inventor: HUIJBREGTS, Laurentia Johanna, 5656 AE Eindhoven (NL); JOHNSON, Mark Thomas, 5656 AE Eindhoven (NL); DELLIMORE, Kiran Hamilton J., 5656 AE Eindhoven (NL); GERHARDT, Lutz Christian, 5656 AE Eindhoven (NL); VAN LIESHOUT, Ron Martinus Laurentius, 5656 AE Eindhoven (NL); PELSSERS, Eduard Gerard Marie, 5656 AE Eindhoven (NL)
(74) Representative: Philips Intellectual Property & Standards

(57) **Abstract**

The present invention relates to sweat sensing and the control of sweat sensors for analyzing sweat uptaken by the sweat sensor (600) from a user's skin. The sweat sensor comprises a data receiving unit (602) configured for receiving medical data about the user. A measurement unit (603) is provided which is configured for carrying out based on the sweat uptaken by the sensor, at least one of a sweat rate measurement and a sweat rate proxy measurement. A controller (604) is provided which controls the measurement mode of the measurement unit based on the received medical data about the user. The present invention uses the insight that the sweat sensor should control the mode of the sweat rate proxy measurement depending on the health status of the user. The data receiving unit (602) may be a user interface, a communication interface condition to a database, or a device for measuring the health status of the user directly within the sensor. Measuring the health status can be done, for example, by an SpO₂ sensor, a transcutaneous CO₂ sensor, or an analyte concentration sensor sensing the concentration of the analyte in the uptaken sweat. Since the sweat rate (proxy) measurement mode is adapted to the medical status of the user, the sweat analysis is more accurate and provides analysis results which are clinically more reliable, as will be explained in more detail hereinafter.

## Description

### FIELD OF THE INVENTION

The present invention relates to sweat analysis by wearable sensors. In particular, the present invention relates to a sweat sensor for analyzing a user's sweat uptaken from a user's skin into the sweat sensor, a method of controlling a measurement mode of a wearable sweat sensor, a computer program element, and a use of medical data about a user of a wearable sweat sensor in adapting a measurement mode of the sweat sensor.

### BACKGROUND OF THE INVENTION

Sweat is a non-obtrusively accessible bio-fluid containing physiologically and metabolically rich information. In fact, sweat has many of the same analytes, i.e. biomarkers, chemicals and/or solutes, and analyte concentrations as found in blood and interstitial fluids. Sweat sensing is a non-invasive, continuous and prolonged monitoring of biomarkers, i.e. analytes, in sweat that can give information about health and/or well-being. There is therefore a demand to measure the most meaningful concentration of biomarkers in sweat, for example for monitoring dehydration, stress, sleep, children's health and in perioperative monitoring. This most meaningful concentration is the one existing when the sweat is produced by the sweat glands, preferably directly above the upper coiled duct on the skin.

Some examples of clinically relevant components of sweat are Na⁺, Cl⁻ and/or K⁺ to monitor dehydration, lactate as early warning for inflammation, glucose for diabetics and neonates and cortisol for sleep and stress monitoring, for example.

The following table shows a non-exhaustive list of biomarkers known to be present in sweat, underlining the promise and relevance of sweat sensing to a diversity of applications. In the table, the term LOD stands for required limit of detection.

| **biomarker** | **LOD** | **applications** | **status** |
|---|---|---|---|
| Na⁺, K⁺, Cl⁻ | 10 mM | dehydration monitoring; Cystic Fibrosis diagnosis; | CF diagnosis from sweat in NICU clinical practice |
| lactate | 10 mM | pressure ischemia detection; post-operative monitoring | predictive ischemia marker shown; only temporal correlation with blood shown |
| urea | 1 mM | skin conditions; dehydration monitoring | hypothesis: urea content in sweat keeps skin in good condition |
| ethanol | 1 mM | alcohol use monitoring | on-skin monitoring used in practice |
| ammonium | 1 mM | metabolic disorders | absolute correlation to blood shown |
| glucose | 0.1 mM | diabetes | temporal correlation to blood shown |
| cortisol | 10⁻⁴ mM | stress monitoring | absolute correlation to blood shown |

Further biomarkers known to be present in apocrine sweat comprise proteins, in particular apocrine secretion odor-binding proteins 1 and 2 (ASOB1 and ASOB2), carbohydrate, ferric ions, lipids, steroids, sialomucin and cathelicidin, wherein proteins and sialomicin have been shown to be present in eccrine sweat as well.

The concept of using sweat for non-invasive access to biomarkers and solutes in blood is not new, with clinical work showing promising results as far back as the 1940s and 1950s. However, until now an impactful application of sweat analysis has been limited mainly to cystic fibrosis diagnostics, and testing for drugs of abuse and alcohol. This is due to the fact that using sweat as a clinical sample for sweat analysis requires a minimum quantity of sweat. Sweat sampling, however, is compromised by sample evaporation and lack of appropriate sampling devices and a proper normalization of a sampled volume.

Furthermore, since a part of the analytes in sweat is reabsorbed by the sweat duct before it reaches the skin surface, the concentration measured on the surface of the skin may not be meaningful enough for a good diagnosis. In fact, the development of reliable sweat sensing has been hampered by several issues: First of all, results from sweat sensing have been highly variable. Secondly, the correlation between blood and sweat values appears to be lacking for various biomarkers. Thirdly, the focus of sweat sensing has been on sensors, not on reliable and robust collection methods for the minute amounts of sweat produced.

US 2018/160951 A1 discloses methods and systems for defining physiological states using sweat-sensing devices. An output of sweat-sensing devices is defined by identifying a set of sweat sensor values. Each sweat sensor value of the set of sweat sensor values includes an electrical-signal value produced by a sensor. For each sweat sensor value of the set of sweat sensor values, the electrical-signal value of the sweat sensor value is translated into a voltage value thereby generating a translated sweat sensor value. Each translated sweat sensor value is calibrated by transforming, upon applying a hardware correction value to the translated sweat sensor value, the voltage value into a physiological value. The calibrated sweat sensor values are aggregated and the calibrated sweat sensors values are then modified using received environmental inputs. An output is then generated based on the modified sweat sensor values.

WO 2018/057695 A1 discloses a device for sensing a biofluid, which includes at least one analyte-consuming sensor for measuring at least a first analyte concentration of an analyte in the biofluid and at least one additional component. The at least one additional component maintains analyte-consuming sensor measurements within 20% of the first concentration measurement if a biofluid sample flow rate is less than or equal to 2 times a first biofluid sample flow rate measurement as measured by the device. A method for sensing a biofluid includes measuring a first analyte concentration of an analyte in the biofluid using an analyte-consuming sensor, measuring a first biofluid sample flow rate, and maintaining a subsequent analyte concentration measurement within 20% of the first analyte concentration measurement when a subsequently measured biofluid flow rate is less than or equal to 2 times the first biofluid sample flow rate.

EP 3 364 183 A1 discloses devices and methods to incorporate suspension-based, i.e., hydrogel-based and thixotropic compound-based, ion-selective electrodes and reference electrodes into a wearable sweat sensing device. Embodiments of this device are configured to monitor sweat electrolyte concentrations, trends, and ratios under demanding use conditions. The accompanying method includes use of the disclosed device to track fluid and electrolyte gain and loss in order to produce an electrolyte estimate, such as a sweat electrolyte concentration, a sweat electrolyte concentration trend, a sweat rate, or a concentration ratio between a plurality of electrolytes.

WO 2016/138087 A1 discloses a two-way communication means between a sweat sensing device and a user; at least one means of activating, deactivating, controlling the sampling rate, and controlling the electrical power applied to a particular sweat sensor or group of sensors; a means of isolating a sweat sensor from sweat until needed; a means of selectively stimulating sweat for a particular sweat sensor or group of sensors to manage sweat flow or generation rate; a means of monitoring the power consumption of a sensor device, individual sensors or groups of sensors; a means of monitoring an individual sweat sensor or group of sensors for optimal performance; a means of monitoring whether a sweat sensing patch is in adequate proximity to a wearer's skin to allow device operation; and the ability to use aggregated sweat sensor data correlated with external information to enhance the device's management capabilities.

### SUMMARY OF THE INVENTION

The inventors of the present invention have found that it is advantageous to use the rate of sweat production, i.e. the sweat rate of the user of the sweat sensor, as a variable to define how close the measured biomarker level is to the original value, i.e. the value of the biomarker as released by the sweat gland. This finding exploits the fact that there is a limited reabsorption rate and hence that as the sweat production increases relatively less biomarkers will be reabsorbed. As such, the measured biomarker level becomes closer to the actual level (as secreted by the coiled tubular part of the glands; rather similar to concentrations in blood) as the sweat production increases. The sweat rate can be measured directly with e.g. a flow sensor. One embodiment to estimate the sweat rate is to use the pH or the analyte concentration of the sweat as a proxy measurement for the sweat rate, but any other sweat rate proxy measurement can be used as well.

The inventors have now also found that for several classes of users and patients the sweat rate measurement/proxy measurement of the sweat rate can be disturbed by characteristics of the user's condition, which modify sweat characteristics independent of the sweat rate.

It is, inter alia, an object of the present invention to provide for an improved sweat sensor for analyzing a user's sweat uptaken by the sensor and for an improved method of controlling a measurement mode of a sweat sensor.

The invention is defined by the subject-matter of the independent claims. Further embodiments and advantages of the invention are defined by the dependent claims. Furthermore, it shall be noted that all embodiments of the present invention concerning a method might be carried out with the order of the steps as described, nevertheless this has not to be the only and essential order of the steps of the method as presented herein. The method disclosed herein can be carried out with another order of the disclosed steps without departing from the respective method embodiment, unless explicitly mentioned to the contrary hereinafter.

Technical terms are used by their common sense. If a specific meaning is conveyed to certain terms, definitions of terms will be given in the following in the context of which the terms are used.

It should be noted that the term "user" as disclosed herein shall be understood to comprise human beings as well as animals. Thus, the sweat sensor as well as the method of the present invention cannot only be applied for human beings, but also for animals. In particular, livestock monitoring is a valuable application of the present invention.

According to a first aspect of the present invention, a sweat sensor for analyzing sweat uptaken by the sweat sensor from a user's skin is presented. A sweat sensor comprises a data receiving unit configured for receiving medical data about the user. The sweat sensor further comprises a measurement unit configured for carrying out based on the sweat uptaken by the sensor, at least one of a sweat rate measurement and a sweat rate proxy measurement. Furthermore, the sweat sensor comprises a controller which is configured for adapting a measurement mode of the measurement unit based on the received medical data about the user. Preferably, the adaptation of the measurement mode is carried out and/or caused by the controller for the next measurement cycle of the sweat sensor.

In other words, the present invention suggests an adaptation of the measurement mode of the sweat rate depending on medical data about the user.

Hence, the sweat sensor presented herein controls the mode of the sweat rate measurement/the sweat rate proxy measurement depending on the health status of the user. This sweat sensor not only comprises that the sweat rate proxy measurement is adapted accordingly by the controller, but also covers the adaptation of a direct measurement of the sweat rate, for example by a flow sensor, which measurement mode adaptation is controlled by the controller of the sweat sensor presented herein.

Since with the sweat sensor of the present invention the measurement mode of the measurement unit is adapted to the individual user of the sweat sensor, an improved analysis of sweat is facilitated. In particular, since the inventors have realized that for several classes of users/patients, the proxy measurement of sweat is disturbed by characteristics of the patient's condition which modify the sweat characteristics independent of the sweat rate, the adaptation of the measurement mode leads to a more accurate analysis of the sweat uptaken by the sweat sensor.

By adapting the sweat rate (proxy) measurement mode to the medical status of the user of the sweat sensor, the sweat analysis is more accurate and provides sweat analysis results which are clinically more reliable. This will be explained in more detail hereinafter.

As was described before, the sweat sensor can carry out a sweat rate proxy measurement. In this context the sweat sensor may measure/determine the sweat rate by a measurement of the osmolality. In principle, the body of the user does not want to get rid of all ions and therefore tries to reabsorb in the sweat duct while the sweat is transported from the sweat gland, via the sweat duct, to the skin surface. However, if the sweat rate is high, this re-absorption cannot fully take place. The analytes will only to a lesser extent get reabsorbed. Therefore, the concentration of certain analytes, in particular Na⁺ and Cl⁻, in the sweat are high for high sweat rates and low for lower sweat rates. This concentration, i.e. osmolality, can thus be measured as proxy/surrogate for the sweat rate measurement. In that sense it is a proxy measurement of the sweat and can be used in an exemplary embodiment example of the present invention. Similarly, pH can be used, just to mention one other example of a sweat rate proxy measurement. Another surrogate/proxy measurement for sweat rate is the galvanic skin response, i.e. GSR, as mentioned hereinbefore and hereinafter.

Therefore, the determination of the sweat rate of the user may be a direct sweat rate measurement, for example by using flow sensors, as described before and hereinafter. Alternatively or in addition, also a surrogate measurement like the measurement of the concentration of certain analytes, in particular Na⁺ and Cl⁻, can be used. More details about the physiological background can be found in the scientific article of Z. Sonner et al. "The microfluidics of the eccrine sweat gland, including biomarker partitioning, transport, and biosensing implications" published in Biomicrofluidics 9, 031301.

The sweat sensor can be configured to compare the received medical data with reference data to determine, which measurement mode is the best match for the individual situation, i.e. for the medical data about the user received. Such reference data may be stored in the sweat sensor, but may also be accessed by the sweat sensor via a communication connection to an external storage device, like e.g. an EMR (electronic medical record) or a cloud storing the reference data.

It should be noted that the term "data receiving unit" shall be understood broadly and shall cover any unit that is able to receive and/or generate medical data about the user. In a particular embodiment, the data receiving unit is realized as a user interface, like e.g. a touch screen, or e.g. as a communication interface connecting wirelessly to a medical database comprising patient data, or as a device for measuring the health status of the user like for example a transcutaneous CO₂ sensor or an SpO₂ sensor. In other words, there are several ways how the sweat sensor can acquire, retrieve or generate the medical data about the user, based on which the adaptation of the measurement mode of the measurement unit is caused subsequently.

For example, the data receiving unit could retrieve information on whether the patient has COPD or asthma from the patient profile in a database, such as an EMR. Details about the non-limiting example in which the user has respiratory problems, as well as about the corresponding flow rate measurement mode adaptation carried out by the sweat sensor will be explained in detail hereinafter.

In another embodiment, the data receiving unit is embodied as for example in a button or a touch screen, by which e.g. a nurse could manually indicate that the patient has particular problems or has a particular health status. Such input data are then considered as the medical data about the user as described herein.

The data receiving unit may also be an interface that is coupled to for example a transcutaneous CO₂ sensor as they are known in the prior art from e.g. WO 2019/121395. Alternatively, the data receiving unit could be coupled to an SpO₂ sensor. Such embodiments can be of particular importance of users that have respiratory problems like COPD or asthma. Such patient groups have an elevated CO₂ level, which can be detected by means of such a CO₂ sensor. This will be described in more detail hereinafter in the context of a particular embodiment.

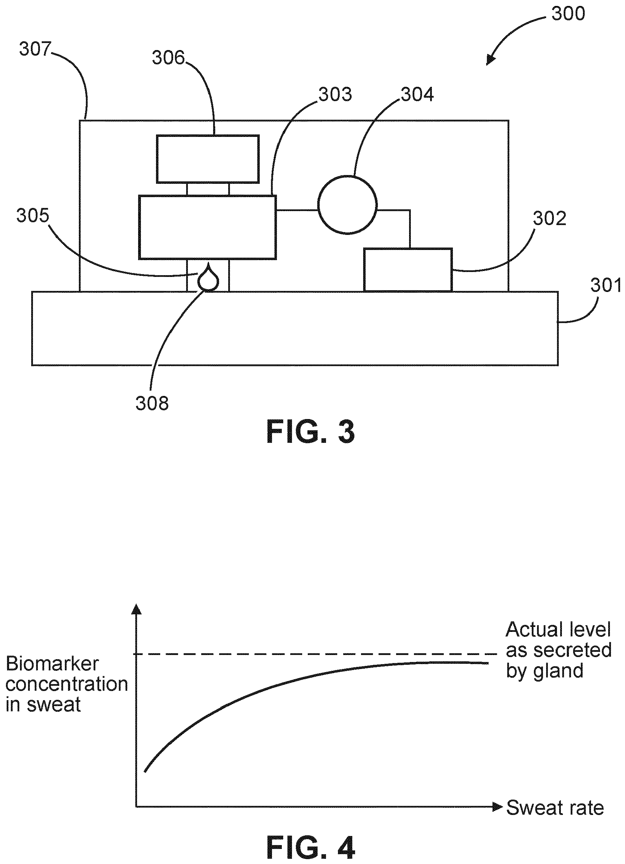

In general, it should be understood that the data receiving unit of the sweat sensor of the present invention may retrieve the medical data about the user from an external entity, but it is also possible that the data receiving unit is coupled to a sensor or comprises a sensor which itself generates the medical data about the user. For example, in the exemplary embodiment described in the context of Fig. 3, the data receiving unit comprises the second pH sensor 302 which is configured for determining a pH value of the user's skin surface as the medical data about the user that are used for the adaptation of the measurement mode of the measurement unit 303. This will be explained in more detail hereinafter in the context of the embodiment of Fig. 3.

Moreover, the term "measurement unit" shall be understood broadly and shall cover any unit that is capable of carrying out a sweat rate measurement or a sweat rate proxy measurement.

In the context of the present invention, the sweat rate proxy measurement shall be understood as a measurement on the sweat that is generated by the user, which is indicative for the sweat rate. Exemplary embodiments of sweat rate proxy measurements are measuring the pH of the user's skin, measuring the pH of the uptaken sweat or measuring the concentration of an analyte in the uptaken sweat. However, other sweat rate proxy measurements are also included by the sweat sensor of the present invention.

In other words, the sweat sensor of the present invention offers an adapted mode selection of sweat rate (proxy) measurement, in particular sweat pH and analyte concentration. The sweat sensor can enter different usage modes depending on specific patient disease characteristics and/or environmental changes associated with the wear time of the sweat sensor on the skin. Exemplary embodiments are a pH proxy mode, an analyte concentration or disease-specific analyte concentration mode. This will be explained in more detail hereinafter.

The mode adaptation carried out by the sweat sensor as presented herein can be based on, for example,
- the modification of the calibration between pH or analyte concentration and sweat rate,
- the correction of the pH depending on the contact time of the sweat sensor with the human skin,
- the addition of an extra physiological measurement in order to re-calibrate the measurement unit of the sensor for the specific user,
- the choice of either measuring pH or analyte concentration by the measurement unit depending on the patient class/medical data about the user,
- the choice of a different or additional sweat rate proxy measurement, and
- the abandonment of the sweat rate proxy measurement and the reversion/activation of a flow sensor directly measuring the sweat rate of the user of the sweat rate.

This will be explained in more detail in the context of exemplary embodiments hereinafter. Note that the mode adaptation carried out by the sweat sensor can be purely automatic.

As will become apparent from the following disclosure, such a sweat sensor is of particular benefit when the sweat sensor is configured and used for estimating a biomarker concentration in the sweat as released by a sweat gland of the user. In such an embodiment of the present invention, the sweat sensor comprises a biomarker concentration sensor configured for determining a concentration value of the biomarker in sweat uptaken by the sensor. Exemplary, non-limiting examples of such biomarkers measured by the biomarker concentration sensor are Na⁺, K⁺, Cl⁻, lactate, urea, ethanol, ammonium, glucose, cortisol, or any combination thereof.

In particular, embodiment, the sweat sensor is configured for correcting the measured biomarker concentration in order to compensate for reabsorption effects that take place between the secretion of the sweat by the gland and the up taking of said sweat by the sweat senor on the skin of the user. Such a compensation carried out by the sweat sensor will be referred to hereinafter as the estimation of the biomarker concentration in the sweat as released by a sweat gland.

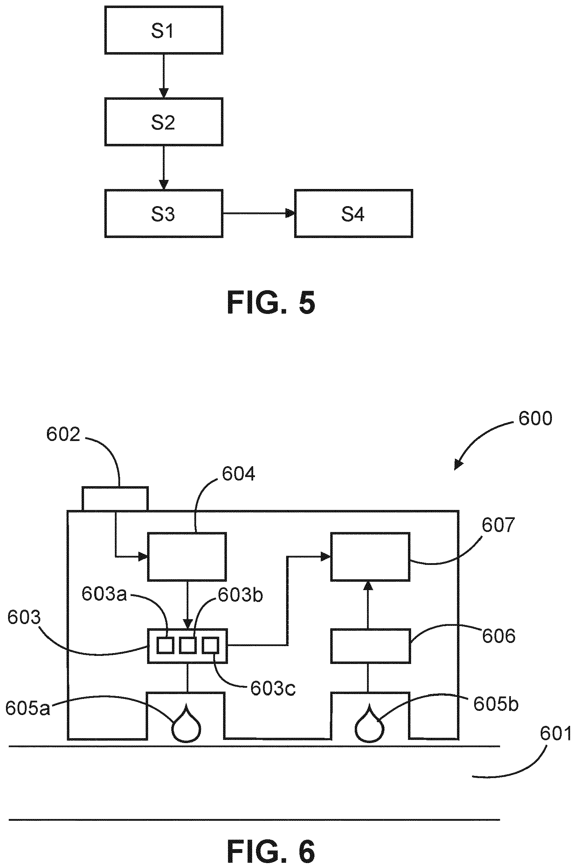

Thus, according to an exemplary embodiment, the sweat sensor is configured for estimating the biomarker concentration in the sweat as released by the sweat gland of the user. The biomarker can be at least a biomarker selected from the previously mentioned list. More details will be explained hereinafter, for example in the context of the non-limiting embodiment shown in Figure 6.

The sweat sensor may use for said estimation the biomarker concentration measured by the biomarker concentration sensor and the sweat rate determined by the sweat rate measurement and/or by the sweat rate proxy measurement carried out by the sweat sensor. In this way, the sweat sensor takes into account reabsorption effects of biomarkers in the sweat between the secretion of the sweat within the skin and the location and time of taking up the sweat by the sensor on the skin. Since also the sweat rate (proxy) measurement mode is adapted to the medical status of the user as suggested with the present invention, the sweat analysis is more accurate and provides analysis results, which are clinically more reliable.

According to an exemplary embodiment of the present invention, the measurement unit of the sweat sensor comprises at least two of the following sensors, preferably all three of the following sensors. These three sensors are:
a) a flow sensor configured for carrying out the sweat rate measurement and for determining a sweat rate of the user,
b) a pH sensor configured for determining the pH value of the skin surface or the uptaken sweat as a sweat rate proxy measurement, and
c) an analyte concentration sensor configured for determining a concentration of an analyte in the uptaken sweat, wherein the concentration of the analyte in the sweat is indicative for a sweat rate of the user, and
wherein the controller is configured for selecting/activating, for the next measurement cycle of the sweat sensor, either the flow sensor or the pH sensor or the analyte concentration sensor based on the received medical data about the user.

In other words, the controller is configured for selecting one of the at least two sensors that are comprised by the sweat sensor, preferably it also means that the controller is configured for deactivating the other one of the two sensors.

In preferred embodiments, the sweat rate is determined by means of measuring fluid flow of the uptaken sweat using a state of the art flow sensor. This can for example entail the measuring of a temperature difference.

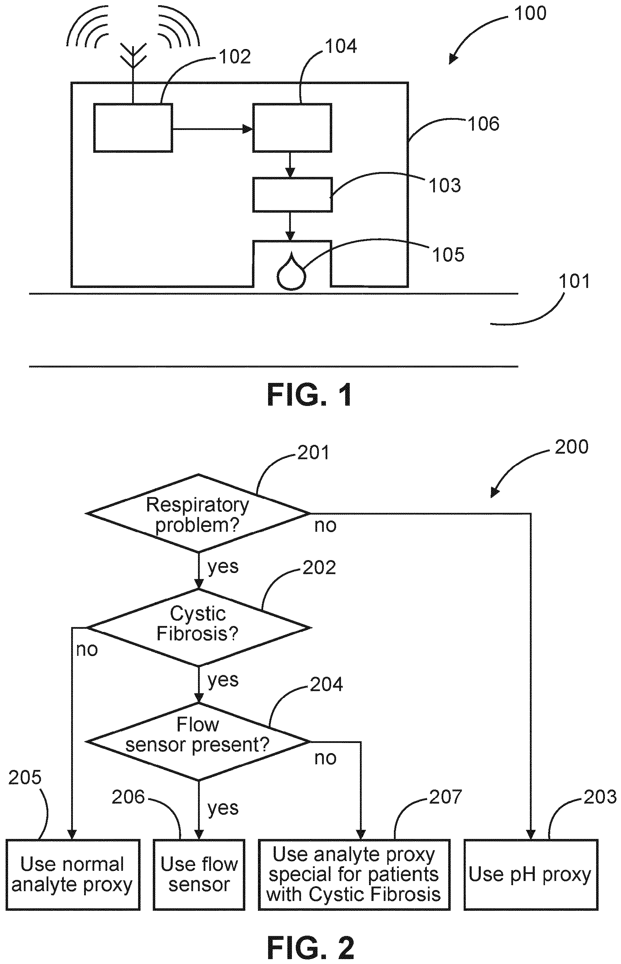

It should explicitly be noted, that any combination of the three previously mentioned sensors, the flow sensor, the pH sensor and the analyte concentration sensor are disclosed herewith. In other words, the sweat sensor may comprise a flow sensor and a pH sensor in a first embodiment, it may comprise a flow sensor and an analyte concentration sensor in a second embodiment, it may comprise a pH sensor and an analyte concentration sensor in a third embodiment, and it may comprise all three sensors according to a fourth embodiment. Particular combinations of these three sensors will be described in the context and elucidated with embodiments detailed hereinafter, see e.g. the embodiments of Figures 1, 3 and 6.

Depending on the medical data about the user of the sweat sensor, the controller selects/activates, for the next measurement cycle of the sweat sensor, which sensor is the most appropriate one. In other words, depending on the medical circumstances of the user, the measurement of the sweat rate by the sweat sensor is advantageously adapted.

The sweat sensor can be configured to compare the received medical data with reference data to determine, which measurement mode is the best match for the individual situation, i.e. for the medical data about the user received. Such reference data may be stored in the sweat sensor, but may also be accessed by the sweat sensor via a communication connection to an external storage device, like e.g. an EMR or a cloud storing the reference data.

Note that in the sweat sensor there are preferably an analyte concentration sensor as well as a biomarker concentration sensor. The analyte concentration sensor measures a concentration that is indicative for the sweat rate and the biomarker concentration sensor measures the concentration of the biomarker of interest, which one can only interpret properly if one knows a proxy for the sweat rate or the sweat rate. The analyte concentration sensor is only activated and used if the analyte concentration is a useful proxy for the sweat rate. The biomarker concentration sensor is absent if there is no interest in a biomarker concentration from a medical perspective, and in this case, the sweat sensor is used to just determine the sweat rate. However, as will be described in detail hereinafter for particular embodiments, for example the embodiment shown in Fig. 6, the present invention can be advantageously used for correcting a determined value of a biomarker concentration based on a measured flow rate, a measured pH value, a measured rate of a change of a pH value of sweat on a skin of the user or based on a measured concentration of an analyte in the sweat that is indicative for the sweat rate.

In other words, in this embodiment of the present invention, the sweat sensor uses the sweat rate as a variable to define how close the measured biomarker concentration is to the original value. This embodiment exploits the fact that there is a limited reabsorption rate and hence as the sweat production increases relatively less biomarkers will be reabsorbed in this sweat duct in the skin of the user. This effect is depicted in Fig. 4. As such, the measured biomarker concentration becomes closer to the actual level, i.e. as secreted by the coiled tubular part of the glands, rather similar to concentrations in blood, as the sweat rate increases. One approach to estimate the sweat rate is, as has been explained before, to use the pH or the analyte concentration of the sweat as a proxy for the sweat rate.

Since the inventors of the present invention have advantageously found that for several classes of patients the proxy measurement of sweat is disturbed by characteristics of the patient, in particular the patient's condition, which modify the sweat characteristics independent of the sweat rate, the adaptation of the measurement mode based on the medical data about the user improves the sweat analysis according to the present invention.

According to an exemplary embodiment of the present invention, the controller is configured for activating the analyte concentration sensor or the flow sensor for the next measurement cycle of the sweat sensor if the received medical data indicate a respiratory malfunction, a kidney dysfunction, or a particular form of acidosis of the user.

It should be noted, that these received medical data may be received from an external entity, but could also be directly measured by the sensor itself, as was described hereinbefore with the example of an SpO₂ sensor, a transcutaneous CO₂ sensor and/or an analyte concentration sensor.

As soon as the sweat sensor has received, i.e. acquired or generated, the medical data that indicate that the user is suffering from respiratory problems, a kidney dysfunction or a particular form of acidosis, the sensor refrains from using the pH as proxy for the sweat rate and will use one of the analyte concentration sensor or the flow sensor for the next measurement cycle. This is done by the controlling of the measurement unit carried out by the controller with corresponding control signals that the controller generates and sends to the measurement unit. For example, the controller could adapt/activate the measurement mode of the measurement unit to measure the concentration of sodium ions.

In this embodiment, the sensor is able to adapt the measurement mode for users or patient groups with elevated CO₂ levels, like users suffering from respiratory problems e.g. COPD, or Asthma. The proposed use of pH as proxy of sweat rate was based on the fact that the sweat duct is able to acidify sweat; although the primary sweat in the secretory coil is usually slightly alkaline, the final sweat leaving the skin is acidic and the pH-value depends on the sweat rate. However, if the primary sweat would have a pH that differs from normal, this will also influence the pH-value of the final sweat and therefore a look-up table or function describing the relationship between the pH of sweat and the sweat rate wouldn't hold anymore. This is the case when the blood has a lower pH than normal, i.e. for acidosis. One major cause for acidosis is hypercapnia, i.e. an elevated CO₂ blood concentration, due to respiratory problems.

There are several ways how the sweat sensor could detect that the wearer/user of the sweat sensor is suffering from respiratory problems.

The sweat sensor could retrieve information on whether the patient has COPD or asthma from the patient profile in a database, such as an EMR (Electronic Medical Record). Also information on medications that in elevated doses can cause acidosis by disrupting the body's acid-base equilibrium are also extracted from the EMR. These include drugs such as biguanides (e.g. metformin), ethanol, methanol, antiretroviral drugs, cyanide, theophylline, cocaine, simvastatin, salicylates, paracetamol (acetaminophen), lactulose, propylene glycol epinephrine, and norepinephrine.

The sweat sensor could contain a button or touch screen, by which e.g. a nurse could manually indicate that the patient has respiratory problems.

The sweat sensor could contain or be coupled to a transcutaneous CO₂ sensor; such sensors are known in prior art, e.g. from Radiometer, are placed on the skin and contain a CO₂-permeable membrane, for example described in WO2019121395. The sweat sensor could contain or be coupled to an SpO₂ sensor. Although this is not a direct indicator of having a high CO₂ concentration in the blood, a low SpO₂ value and a high CO₂ concentration are often correlated. The advantage of using an SpO₂ sensor instead of a CO₂ sensor is that it fits much better in the workflow of the nurse.

The sweat sensor could contain a biomarker sensor which measures the lactate and pyruvate concentrations in sweat, or alternatively the ratio between these two biomarkers. A rise in the concentration of lactate in sweat out of proportion to the level of pyruvate, i.e., "excess lactate" is an indicator of metabolic acidosis. This is an indicator of fermentation due to anaerobic metabolism occurring in muscle cells. In poorly-oxygenated tissue cells that contain mitochondrion, pyruvate is preferentially converted to lactate instead of being metabolized to carbon dioxide and water in mitochondria via two integrated metabolic pathways: the citric acid cycle and oxidative phosphorylation.

As soon as the sweat sensor has detected that the wearer is suffering from respiratory problems based on the received medical data about the user, the sweat sensor would refrain from using the pH as proxy for sweat rate and will use one of the afore-mentioned modified measurement modes. For example, it could switch to the use of an analyte concentration as sweat rate proxy. For example, the concentration of sodium ions.

It should be noted that this controlling of the measurement unit is based on the insight, that for respiratory malfunctions and metabolic acidosis, the pH sensor is no longer reliable since the body produces too much acid or not enough is removed by the kidneys.

According to another exemplary embodiment of the present invention, the controller is configured for changing a measurement mode of the analyte concentration sensor for the next measurement cycle of the sweat sensor, preferably using a different calibration of the analyte concentration sensor if the received medical data about the user indicate that the user has Cystic Fibrosis. Alternatively, the controller is configured for activating the flow sensor and for deactivating the analyte concentration sensor for the next measurement cycle of the sweat sensor, if the received medical data about the user indicate that the user has Cystic Fibrosis.

When the sweat sensor has received medical data that indicate that the patient has Cystic Fibrosis, the controller could control that the flow sensor is used in at least the next measurement cycle of the sweat sensor, which directly measures the fluid flow of the uptaken sweat and directly determines the sweat rate. Alternatively, the controller could control the measurement unit to use the relationship between Na⁺ and/or Cl⁻ concentrations as function of the sweat rate defined for Cystic Fibrosis patients, which are defined in reference data like e.g. a look-up table within the sensor or external of the sensor. In other words, in such a situation, the controller would cause that a different dependency/curve between the sweat rate and the concentration of these ions within the sweat at the skin surface is used. In particular, a different calibration of the analyte sensor may be used in view of the explanations of Z. Sonner et al. in "The microfluidics of the eccrine sweat gland, including biomarker partitioning, transport, and biosensing implications" published in Biomicrofluidics 9, 031301.

According to another exemplary embodiment of the present invention, the measurement unit comprises at least one pH sensor configured for determining a pH value of the skin surface or of the uptaken sweat as a sweat rate proxy measurement. The controller is configured for causing a continuous pH re-calibration or pH correction of the measurement of the pH sensor if the received medical data indicate that the sweat sensor has been in contact with the user's skin.

According to another exemplary embodiment, the measurement unit comprises a first pH sensor configured for determining the pH value of the uptaken sweat as a sweat rate proxy measurement. The data receiving unit comprises a second pH sensor configured for determining a pH value of a user's skin surface as the medical data about the user. The controller is configured for causing a continuous pH re-calibration or pH correction of a measurement of the first pH sensor based on the determined pH value of the user's skin surface.

These embodiments relate to the correction for skin pH change or ion concentration as a function of wear time /device contact time with human skin and differential pH or ion measurement. The sweat sensor can enter or use a pH re-calibration or correction mode associated with the wear time of the device on the skin.

It is known that the skin pH can dramatically change with wear time of patches due to an effect called occlusion (transepidermal water loss through the skin is impaired and an oxygen deprived microclimate underneath the sensor is created). In this case, the pH and analyte proxy measurements are disturbed by skin occlusion leading to skin pH and microflora changes which alter both ion concentration and pH measurements of fresh sweat from the sweat glands reaching the acidic skin surface. Especially for semi-continuous long term monitoring (3-5 days) of sweat, the change of the skin microflora needs to be taken into account.

To make the sweat rate proxy measurement using pH more reliable and robust to the skin occlusion-induced pH effect, this embodiment proposes compensation or correction of sweat pH measurement by skin pH. The simplest way to measure the true pH of fresh (unmixed) sweat is done by subtracting the skin pH value from the pH of the analyte sweat entering the sweat patch, as is the case of the embodiment shown in Figure 3.

Therefore, in another embodiment similar to the one in Figure 3, the measurement unit comprises at least one analyte concentration sensor for determining the analyte concentration of the uptaken sweat as a sweat rate proxy measurement. The data receiving unit comprises a second analyte concentration sensor for determining a concentration of the analyte on the user's skin surface as the medical data about the user and the controller is configured for causing a continuous concentration re-calibration or analyte concentration correction of the measurement of the first analyte concentration sensor based on the determined analyte concentration value of the user's skin surface.

According to another exemplary embodiment, the controller adapts the measurement mode of the measurement unit by using a different look-up table describing the pH as a function of sweat rate depending on the size of the eccrine sweat glands of the individual user.

The maximum sweat rate is dependent on the glandular size of the eccrine glands. With increased glandular size, a longer coiled tubular body, it is plausible that the pH is increased more steeply as function of sweat rate. Consequently (i) classifying persons as poor, moderate and heavy sweaters and (ii) subsequently selecting a look-up table, related to this classification, describing the pH as function of sweat rate will make the proxy pH for sweat rate more accurate.

According to another exemplary embodiment of the present invention, the sweat rate proxy measurement determines a sweat parameter of the user's sweat uptaken by the sensor which determined parameter is indicative for the sweat rate of the user.

Particular embodiments of the sweat rate proxy measurements are the measurement of pH of the user's skin or of the uptaken sweat, or the measurement of an analyte concentration in the uptaken sweat. Preferred analytes are selected from the group comprising comprising Na⁺, K⁺, Cl⁻, but also other analytes that can be used as a sweat rate proxy can be used.

According to another exemplary embodiment of the present invention, the sweat sensor is configured for measuring a health status of the user thereby generating the medical data about the user. The sweat sensor preferably comprises, for measuring the health status of the user, an SpO₂ sensor, a transcutaneous CO₂ sensor and/or an analyte concentration sensor.

In particular, when the user suffers from respiratory problems like for example COPD or asthma, the measurement of CO₂ and/or SpO₂ can be used to identify such respiratory patients, since they are a patient group with an elevated CO₂ level.

According to another exemplary embodiment of the present invention, the data receiving unit of the sweat sensor comprises a communication interface configured for retrieving the medical data about the user from a database, like for example an electronic medical record (EMR). Additionally or alternatively, the sweat sensor may comprise a user interface, preferably a touch screen, for receiving a manual indication about the user's health status. This indication about the user's health status are the medical data about the user as disclosed herein.

According to another exemplary embodiment of the present invention, the sweat sensor is for estimating a biomarker concentration in the sweat as released by a sweat gland of the user. The measurement unit is configured to obtain at least one of a flow rate, a pH value, a rate of change of pH value of sweat on a skin of the user, and a concentration of an analyte in the sweat that is indicative for the sweat rate. The sweat sensor further comprises a biomarker concentration sensor configured for determining a concentration value of the biomarker in sweat uptaken by the sensor. The sweat sensor further comprises a processing unit configured to correct the determined value of the biomarker concentration according to a correction model for estimating the biomarker concentration as released by the sweat gland based on at least one of the flow rate, the pH value and the rate of change of the pH value of the sweat and the concentration of the analyte in the sweat to correct the biomarker concentration for reabsorption effects related to the flow rate and/or the pH value and/or the rate of change of the pH value, and/or the concentration of the analyte in the sweat.

This embodiment has the advantage to take into account the reabsorption of analytes from sweat as the sweat leaves the sweat duct while estimating the biomarker concentration in the sweat as released by a sweat gland.

Accordingly, effects of physiological mechanisms causing incorrect measurements of analyte concentrations in sweat are reduced or even eliminated by the present invention. In this embodiment, the biomarker concentrations as sensed on a user's skin are corrected with respect to the flow rate at which said sweat has been released by the sweat glands and/or with respect to the pH value the respective sweat has and/or with respect to a rate of change of the sweat's pH value and/or with respect to a concentration of the analyte of the sweat.

The correction model used takes effects of reabsorption, particularly occurring along the entire sweat duct from the sweat gland until the pore, into account. In fact, the correction model is configured to take into account any flow rate, pH value, rate of change of the pH value as well as analyte concentration and the determined biomarker concentration obtained as input in order to correct the biomarker concentration based on said parameters.

Hence, the rate of sweat production is used as a variable to define how close the measured biomarker level is to the original value, i.e. the value when secreted by the gland, which is closest to the level in blood. This exploits the fact that there is a limited reabsorption rate and hence that as the sweat production increases relatively less biomarkers will be reabsorbed. As such, the measured biomarker level becomes closer to the actual level (as secreted by the coiled tubular part of the glands; rather similar to concentrations in blood) as the sweat production increases. This can be seen in Figure 4.

According to an exemplary embodiment, the biomarker concentration in the sweat as released by a sweat gland, which is estimated by the sweat sensor is at least one of Na+, K+, Cl-, lactate, urea, ethanol, ammonium, glucose, cortisol, or any combination thereof.

In a preferred embodiment the sensor is only exposed to sweat above a threshold production level. In this manner the measurement reflects the most reliable level of biomarkers in the sweat.

In the following particular embodiments of the sweat sensor that measures the biomarker concentration with a biomarker concentration sensor are described.

In one embodiment, a sweat production patch with a flow rate sensor and a look-up table is presented. In this embodiment the sweat sensor is embodied as a patch or other wearable device, whereby the sweat sensor is further provided with a sweat flow measurement sensor and a look-up table. The look-up table contains the concentration of a particular biomarker of interest versus the sweat rate, e.g. derived from a set of healthy volunteers.

In another embodiment, a sweat production patch with flow rate sensor and fluid switch is presented. In this embodiment the sweat sensor is configured to continuously record the flow rate of the sweat, but to control the fluid switch to direct the sweat away from the biomarker concentration sensor if the flow rate is below a pre-defined threshold.

In another embodiment, the sweat sensor is a sweat measurement patch with pH sensor. In this embodiment the sweat is collected based upon a second measurement that also indicated that re-adsorption is playing a relatively minor role in the biomarker concentrations in the sweat: namely the pH of the sweat. As such the pH is being used as a proxy for the sweat rate.

In another embodiment, the sweat sensor is a sweat production patch with a pH sensor and fluid switch. The operation of this sensor comprises that the look-up table is now modified to show the influence of pH on the biomarker concentration. Alternatively, the measurement is now triggered to start only when the pH is above a certain threshold level, for example above pH 4, more preferably above pH 4.5, even more preferably above pH 5.

In another embodiment, the sweat sensor sweat production patch with a passive flow rate sensor and fluid switch. In this embodiment it is proposed to use a passive means to measure the sweat flow rate (or change in flow rate) and to trigger the fluid switch to direct the sweat towards the biomarker concentration sensor.

According to another aspect of the present invention, a method of controlling a measurement mode of a sweat sensor is presented. The method comprises the steps of receiving medical data about the user by the sweat sensor, wherein sweat sensor comprises a measurement unit configured for carrying out, based on the sweat uptaken by the sensor, at least one of a sweat rate measurement and a sweat rate proxy measurement. The method further comprises the step of generating a control signal based on the received medical data about the user and causing, with the generated control signal, the adaptation of the measurement mode of the measurement unit of the sweat sensor for the next measurement cycle of the sweat sensor.

According to another exemplary embodiment of the present invention, the measurement unit comprises at least two sensors, preferably all three sensors, of:
a) a flow sensor configured for carrying out the sweat rate measurement and for determining a sweat rate of the user,
b) a sweat pH sensor configured for determining the pH value of the uptaken sweat as a sweat rate proxy measurement, and
c) an analyte concentration sensor configured for determining a concentration of the analyte in the uptaken sweat, wherein the concentration of the analyte in the sweat is indicative for a sweat rate of the user.

The method further comprises the step of selecting, for the use in the next measurement cycle of the sweat sensor, either the flow sensor or the sweat pH sensor or the analyte concentration sensor based on the received medical data about the user.

In other words, the method presented herein comprises an adaptation of the measurement mode which is carried out by a controller for the next measurement cycle of the sweat sensor.

According to another aspect of the present invention, the use of medical data about a user of a sweat sensor in adapting, for a next measurement cycle of the sweat sensor, a measurement mode of a measurement unit of the sweat sensor is presented. The measurement unit of the sweat sensor carries out at least one of a sweat rate measurement and a sweat rate proxy measurement. The sweat rate proxy measurement determines a sweat parameter of the user's sweat, which is indicative for the sweat rate of the user.

According to another aspect of the present invention, a computer program element is presented, which when being processed by a processor, causes the processor to carry out the following steps: receiving medical data about a user of a sweat sensor by the sweat sensor comprising a measurement unit configured for carrying out, based on the sweat uptaken by the sensor, at least one of a sweat rate measurement and a sweat rate proxy measurement. A further step of the processor carrying out this method is the generation of a control signal based on the received medical data about the user and causing, with the control signal, an adaptation of a measurement mode of the measurement unit of the sweat sensor for a next measurement cycle of the sweat sensor.

The computer program element may be part of a computer program, but it can also be an entire program by itself. For example, the computer program element may be used to update an already existing computer program to get to the present invention. The computer readable medium storing the computer program element may be seen as a storage medium, such as for example, a USB stick, a CD, a DVD, a data storage device, a hard disk, or any other medium on which a program element as described above can be stored.

These and other aspects of the invention will be apparent from and elucidated with reference to the embodiments described hereinafter. In the following drawings.

### BRIEF DESCRIPTION OF THE DRAWINGS

Fig. 1 schematically shows a sweat sensor for analyzing sweat uptaken by the sweat sensor from a user's skin according to an exemplary embodiment of the present invention.
Fig. 2 schematically shows a decision tree/flow diagram carried out in a method or by a sweat sensor according to an exemplary embodiment of the present invention.
Fig. 3 schematically shows a sweat sensor according to another exemplary embodiment of the present invention.
Fig. 4 schematically shows the dependency of a measured biomarker concentration in sweat from the sweat rate and the relation to the actual level as secreted by a gland.
Fig. 5 schematically shows a method of controlling a measurement mode of a sweat sensor according to an exemplary embodiment of the present invention.
Fig. 6 schematically shows a sweat sensor according to another exemplary embodiment of the present invention.

### DETAILED DESCRIPTION OF THE INVENTION

Fig. 1 shows a sweat sensor 100 for analyzing sweat uptaken by the sweat sensor 100 from a user's skin 101. The sweat sensor 100 comprises a data receiving unit 102 configured for receiving medical data about the user. In the embodiment shown in Fig. 1, the data receiving unit is realized as a communication interface 102 configured for wirelessly retrieving the medical data about the user from a database, e.g. an electronic medical record (EMR). The sweat sensor 100 further comprises a measurement unit 103 which is configured for carrying out, based on the uptaken sweat 105, a sweat rate measurement and/or a sweat rate proxy measurement. The sensor 100 further comprises a controller 104 which is configured for adapting the measurement mode of the measurement unit 103 based on the received medical data about the user, which was received by data receiving unit 102. Since the inventors of the present invention have advantageously found that for several classes of users/patients the proxy measurement of sweat is disturbed by characteristics of the patient, in particular the patient's condition, which modify the sweat characteristics independent of the sweat rate, the adaptation of the measurement mode based on the medical data about the user improves the sweat analysis according to the present invention.

The measurement unit 103 may for example comprise a flow sensor, a pH sensor and an analyte concentration sensor. Depending on the received medical data about the user, the controller activates and deactivates the sensors of the measurement unit or e.g. adapts their calibration accordingly. In particular, the sweat sensor 100 for example, when the sensor 100 detects that the user is suffering from respiratory problems, the sensor 100 refrains from using the pH as a sweat rate proxy and uses one of the aforementioned approaches for the sweat rate measurement/sweat rate proxy measurement for the next analytical cycle.

For example, the controller 104 could switch the measurement mode of the measurement unit 103 to the use of the analyte concentration as sweat rate proxy, for example, the concentration of sodium ions. However, in case the sensor has determined that the patient has Cystic Fibrosis, it could instead use the flow sensor of the measurement unit 103. Yet another option is that the controller controls the measurement mode of the measurement unit 103 to use the relationship between Na⁺ and/or Cl⁻ concentrations as function of sweat rate defined for Cystic Fibrosis patients. Therefore, another calibration curve of the analyte concentration sensor can be used and the controller 103 can initiate the use of such a different calibration curve based on the received medical data that were received by the unit 102.

In case the received medical data specify that the user has a particular size of eccrine sweat glands, the controller 103 can also use another look-up table, related to this user classification regarding the size of his/her eccrine sweat glands, describing the pH as function of sweat rate. This will make the proxy pH for sweat rate more accurate. In other words, the proxy sweat rate measurement in this embodiment is measuring the pH of the sweat and another calibration curve is used depending on what kind of size the eccrine sweat glands of the individual users have.

Fig. 2 schematically shows a decision tree and flow diagram describing how a controller of a sweat sensor according to an embodiment of the present invention and a method of controlling the measurement mode of a sweat sensor according to yet another embodiment of the present invention work. This decision tree makes use of the insight the inventors had regarding the adaptation of the sweat rate (proxy) measurement mode to the medical status of the user. This makes the sweat analysis more accurate and provides analysis results which are clinically more reliable. In step 201, the controller of the sweat sensor processes the question whether the received medical data indicate any respiratory problems of the user. This step is shown in Fig. 2 by reference sign 201. If the answer is Yes, the controller determines in step 202 whether the received medical data indicate that the user has Cystic Fibrosis. If, however, the answer in step 201 is No, the controller sets the measurement mode of the measurement unit to the usage of the pH sensor for determining the pH value of the skin surface or of the uptaken sweat as a sweat rate proxy measurement. This is done in step 203. If the answer to the question about Cystic Fibrosis is Yes, the controller checks whether a flow sensor is present in step 204. If the question about the Cystic Fibrosis is answered with No, the controller in step 205 sets the measurement mode of the measurement unit to the usage of the normal analyte concentration proxy using the analyte concentration sensor. If in step 204 a flow sensor is present, the controller sets the measurement mode of the measurement unit such that the flow sensor is used for the flow rate measurement. If, however, in step 204 the answer is No, the controller ensures that the measurement unit uses the analyte proxy which is especially calibrated for patients with Cystic Fibrosis in step 207. Details about the different medical data indicating respiratory problems, Cystic Fibrosis and the corresponding suggested measurements, have been described hereinbefore in detail. Such a concept can be used in a sweat sensor as described e.g. for Figures 1, 3 and 6.

According to an another exemplary embodiment, Fig. 3 schematically shows a sweat sensor 300 for analyzing sweat uptaken by the sweat sensor from a user's skin 301. The sweat sensor 300 comprises a data receiving unit 302, which is embodied as a pH sensor. In this embodiment, the pH sensor 302 is configured for determining a pH value of a user's skin surface as the medical data about the user. Therefore, in this embodiment, the sweat sensor 300 itself generates the medical data about the user, namely the measured pH value of the user's skin surface. The sweat sensor 300 further comprises the measurement unit 303, which is configured for carrying out a pH value measurement of the uptaken sweat as a sweat rate proxy measurement. The controller 304 is configured for adapting the measurement mode of the pH sensor 303 or performing a pH correction based on the pH value of the user's skin surface determined by sensor 302, i.e. the controller 304 generates a differential pH measurement signal. The uptaken sweat 305 is guided into a liquid channel 308 which has an opening for uptaking the sweat from the user's skin 301. Sweat sensor 300 further comprises a biomarker concentration sensor 306 which is configured for determining a concentration value of the biomarker of interest in the sweat uptaken by the sensor. The controller 304 is configured for causing a continuous pH re-calibration or pH correction of the measurement of the pH sensor 303 based on the determined pH value of the user's skin. The controller 304 may also take into account the time that the sweat sensor has been in contact with the user's skin for a certain time period. Due to the adaptation of the measurement mode of the sweat sensor 303, a correction of the measured biomarker concentration determined by sensor 306 due to reabsorption effects based on the pH value determined by sensor 303 is more accurate, since the change of pH on the skin surface is taken into account.

In its preferred embodiment, the controller 304 performs a differential pH measurement allowing for a continuous pH re-calibration or pH correction. In this embodiment, one pH sensor/electrode or ion-sensitive field-effect transistor (ISFET) sensitive electrode is placed on the skin, i.e. on skin sensor 302), and the another one sensor inside or in close vicinity of the sweat collection channel, i.e. in-device sensor 303), as shown in Figure 3.

In another embodiment, the sweat sensor 300 uses as the on skin sensor 302 a sensor measuring an ion concentration on the skin, and uses as an in-device sensor 303 a sensor measuring the ion concentration of the sweat collected in the channel of the sensor. In this embodiment, the controller 304 generates an ion concentration measurement signal.

Thus, in the embodiments described herein before based on Figure 3, the sweat sensor compensates or corrects the of sweat pH measurement by the skin pH. The simplest way to measure the true pH of fresh (unmixed) sweat is done by subtracting the skin pH value from the pH of the analyte sweat entering the sweat sensor, e.g. a sweat patch, as is the case of the embodiment shown in Figure 3.

In a variant of these embodiments described in the context of Figure 3, i.e. a device training embodiment using big data, a master curve generated from a large population can be used to make the pH correction. This master curve can be established from datasets of many users of connected sweat sensing devices, i.e. training sensor, which have both the on-skin sensor and in-device sensor integrated in the device. Once the master curve has been generated, new sweat sensors without an on-skin sensor (but only an in-device sensor) can be used and re-calibrated on the fly by making use of the master curve. This training embodiment has the advantage that less components need to be integrated in a sweat sensor (replacement sensor) that might be used beyond the typical wear time of the training sensor (3 days up to 15 days).

Figure 4 shows a graph that explains the dependency of the measured biomarker concentration in sweat from the sweat rate of the user. It also shows the relation to the actual level of said biomarker as secreted by the sweat gland inside the skin. This describes the fact that there is a limited reabsorption rate and hence that as the sweat production increases relatively less biomarkers will be reabsorbed. As such, the measured biomarker level becomes closer to the actual level (as secreted by the coiled tubular part of the glands; rather similar to concentrations in blood) as the sweat production increases.

Figure 5 shows a flow diagram of a method of controlling a measurement mode of a sweat sensor. The method comprises receiving medical data about the user by the sweat sensor, step S1. The used sweat sensor comprises a measurement unit configured for carrying out, based on the sweat uptaken by the sensor, at least one of a sweat rate measurement and a sweat rate proxy measurement. In step S2 a control signal is generated by the controller of the sweat sensor based on the received medical data about the user. Moreover, using the control signal to cause the desired adaptation of the measurement mode of the measurement unit of the sweat sensor for at least the next measurement cycle of the sweat sensor is done in step S3. The measurement unit used comprises at least two sensors, preferably all three sensors, of
a) a flow sensor configured for carrying out the sweat rate measurement and for determining a sweat rate of the user,
b) a sweat pH sensor configured for determining the pH value of the uptaken sweat as a sweat rate proxy measurement, and
c) an analyte concentration sensor configured for determining a concentration of the analyte in the uptaken sweat, wherein the concentration of the analyte in the sweat is indicative for a sweat rate of the user.

And the method further comprises step S4 in which either the flow sensor or the sweat pH sensor or the analyte concentration sensor is selected/activated for use in the next measurement cycle of the sweat sensor based on the received medical data about the user. The control signal is used to cause this selection/activation of the proper sensor.

The method described in Figure 5 can be used, e.g. by the sensor shown in Figure 6, which will now be described in detail.

Figure 6 shows a sweat sensor 600 for analyzing sweat uptaken by the sensor from a user's skin 601. The sweat sensor 600 is configured for measuring and for correcting the measured biomarker concentration in order to compensate for reabsorption effects that take place between the secretion of the sweat by the gland and the uptake of said sweat by the sweat senor on the skin of the user. Sweat sensor 600 comprises a data receiving unit 602 configured for receiving medical data about the user. The data receiving unit is embodied a user interface 602, preferably a touch screen, for receiving a manual indication about the user's health status. As was described before the data receiving unit 602 could also be embodied in several different other ways. The sweat sensor 600 also comprises the measurement unit 603 configured for carrying out, based on the sweat 605a uptaken by the sensor 600 at least one of a sweat rate measurement and a sweat rate proxy measurement. For this purpose, the measurement unit comprises 603 a flow sensor 603a configured for carrying out the sweat rate measurement and for determining a sweat rate of the user, a pH sensor 603b configured for determining the pH value of the skin surface or the uptaken sweat as a sweat rate proxy measurement, and an analyte concentration sensor 603c configured for determining a concentration of an analyte in the uptaken sweat. The concentration of the analyte in the sweat is indicative of a sweat rate of the user. Also a controller 604 configured for adapting the measurement mode of the measurement unit 603 based on the medical data about the user received via user interface 602 is comprised. The controller 604 is configured for selecting, for the next measurement cycle of the sweat sensor and based on the received medical data about the user, either the flow sensor 603a or the pH sensor 603b or the analyte concentration sensor 603c.

Sweat sensor 600 further comprises the biomarker concentration sensor 606 configured for determining a concentration value of the biomarker in sweat 605b uptaken by the sensor. Exemplary, non-limiting examples of such biomarkers measured by the biomarker concentration sensor are Na⁺, K⁺, Cl⁻, lactate, urea, ethanol, ammonium, glucose, cortisol, or any combination thereof. Sweat sensor 600 further comprises the processing unit 607 configured to correct the measured value of the biomarker concentration. Such a correction carried out by the sweat sensor is referred to herein as the estimation of the biomarker concentration in the sweat as released by a sweat gland. The sweat sensor 600, in particular the processing unit 607, uses for said estimation the biomarker concentration measured by the biomarker concentration sensor 606 and the sweat rate determined by the measurement unit 603 carrying out a sweat rate measurement and/or a sweat rate proxy measurement. In this way, the sweat sensor 600 takes into account reabsorption effects of biomarkers in the sweat between the secretion of the sweat within the skin and the uptake into the sensor on the skin. Since also the sweat rate (proxy) measurement mode is adapted to the medical status of the user as was explained, the sweat analysis is more accurate and provides analysis results, which are clinically more reliable.

Where an indefinite or definite article is used when referring to a singular noun, e.g. "a", "an" or "the", this includes a plurality of that noun unless something else is specifically stated.

Other variations to the disclosed embodiments can be understood and effected by those skilled in the art in practicing the claimed invention, from the study of the drawings, the disclosure, and the appended claims. In the claims, the word "comprising" does not exclude other elements or steps. A single processor or other unit may fulfill the functions of several items or steps recited in the claims. The mere fact that certain measures are recited in mutually different dependent claims does not indicate that a combination of these measures cannot be used to advantage. A computer program may be stored/distributed on a suitable medium such as an optical storage medium or a solid-state medium supplied together with or as part of other hardware, but may also be distributed in other forms, such as via the Internet or other wired or wireless telecommunication systems. Any reference signs in the claims should not be construed as limiting the scope of the claims.

Embodiments of the present invention thus relate to sweat sensing and the control of sweat sensors for analyzing sweat uptaken by the sweat sensor 600 from a user's skin. The sweat sensor comprises a data receiving unit 602 configured for receiving medical data about the user. A measurement unit 603 is provided which is configured for carrying out based on the sweat uptaken by the sensor, at least one of a sweat rate measurement and a sweat rate proxy measurement. A controller 604 is provided which controls the measurement mode of the measurement unit based on the received medical data about the user. The present invention uses the insight that the sweat sensor should control the mode of the sweat rate proxy measurement depending on the health status of the user. The data receiving unit 602 may be a user interface, a communication interface condition to a database, or a device for measuring the health status of the user directly within the sensor. Measuring the health status can be done, for example, by an SpO₂ sensor, a transcutaneous CO₂ sensor, or an analyte concentration sensor sensing the concentration of the analyte in the uptaken sweat. Since the sweat rate (proxy) measurement mode is adapted to the medical status of the user, the sweat analysis is more accurate and provides analysis results which are clinically more reliable, as will be explained in more detail hereinafter.

## Claims

1. Sweat sensor (100, 300, 600) for analyzing sweat uptaken by the sweat sensor from a user's skin (101, 301, 601), the sweat sensor comprising
a data receiving unit (102, 302, 602) configured for receiving medical data about the user,
a measurement unit (103, 303, 603) configured for carrying out, based on the sweat uptaken (105, 305, 605a) by the sensor, at least one of a sweat rate measurement and a sweat rate proxy measurement, and
a controller (104, 304, 604) configured for adapting a measurement mode of the measurement unit based on the received medical data about the user.

2. Sweat sensor (600) according to claim 1,
wherein the measurement unit (603) comprises at least two, preferably all three, sensors of:
a) a flow sensor (603a) configured for carrying out the sweat rate measurement and for determining a sweat rate of the user,
b) a pH sensor (603b) configured for determining the pH value of the skin surface or the uptaken sweat as a sweat rate proxy measurement, and
c) an analyte concentration sensor (603c) configured for determining a concentration of an analyte in the uptaken sweat, wherein the concentration of the analyte in the sweat is indicative for a sweat rate of the user, and
wherein the controller is configured for selecting, for the next measurement cycle of the sweat sensor, either the flow sensor or the pH sensor or the analyte concentration sensor based on the received medical data about the user.

3. Sweat sensor according to claim 2,
wherein the controller (604) is configured for activating the analyte concentration sensor (603c) or the flow sensor (603a) for the next measurement cycle of the sweat sensor if the received medical data indicate a respiratory malfunction, a kidney dysfunction, or a particular form of acidosis of the user.

4. Sweat sensor according to claim 2 or 3,
wherein the controller (604) is configured for:
a) changing a measurement mode of the analyte concentration sensor (603c) for the next measurement cycle of the sweat sensor, preferably using a different calibration of the analyte concentration sensor, or for
b) activating the flow sensor (603a) and for deactivating the analyte concentration sensor for the next measurement cycle of the sweat sensor,
if the received medical data about the user indicate that the user has Cystic Fibrosis.

5. Sweat sensor according to any of the preceding claims,
wherein the measurement unit comprises at least one pH sensor configured for determining a pH value of the skin surface or of the uptaken sweat as a sweat rate proxy measurement, and
wherein the controller is configured for causing a continuous pH re-calibration or pH correction of the measurement of the pH sensor if the received medical data indicate that the sweat sensor has been in contact with the user's skin.

6. Sweat sensor according to any of the preceding claims,
wherein the measurement unit comprises a first pH sensor (303) configured for determining the pH value of the uptaken sweat (305) as a sweat rate proxy measurement,
wherein the data receiving unit comprises a second pH sensor (302) configured for determining a pH value of a user's skin surface as the medical data about the user, and
wherein the controller (304) is configured for causing a continuous pH re-calibration or pH correction of a measurement of the first pH sensor (303) based on the determined pH value of the user's skin surface.

7. Sweat sensor according to any of the preceding claims,
wherein the sweat rate proxy measurement determines a sweat parameter of the user's sweat uptaken by the sensor, and
wherein the parameter determined in the sweat rate proxy measurement is indicative for the sweat rate of the user.

8. Sweat sensor according to any of the preceding claims,
wherein the sweat rate proxy measurement is a measurement of pH of the user's skin or of the uptaken sweat, or is a measurement of an analyte concentration in the uptaken sweat.

9. Sweat sensor according to claim 8,
wherein the analyte is selected from the group comprising Na⁺, K⁺, Cl⁻, lactate, urea, ethanol, ammonium, glucose, cortisol, a protein, carbohydrate, a ferric ion, a lipid, a steroid, or any combination thereof.

10. Sweat sensor according to any of the preceding claims,
wherein the sweat sensor is configured for measuring a health status of the user thereby generating the medical data about the user, and
wherein the sweat sensor preferably comprises, for measuring the health status of the user, an SpO₂ sensor, a transcutaneous CO₂ sensor and/or an analyte concentration sensor.

11. Sweat sensor according to any of the preceding claims,
wherein the data receiving unit of the sweat sensor comprises:
a communication interface (102) configured for retrieving the medical data about the user from a database, e.g. an electronic medical record (EMR), and/or
a user interface (602), preferably a touch screen, for receiving a manual indication about the user's health status.

12. Sweat sensor (600) according to any of the preceding claims for estimating a biomarker concentration in the sweat as released by a sweat gland of the user,
wherein the measurement unit is configured to obtain at least one of a flow rate, a pH value, a rate of change of pH value of sweat on a skin of the user, a concentration of an analyte in the sweat that is indicative for the sweat rate,
the sweat sensor further comprising
a biomarker concentration sensor (606) configured for determining a concentration value of the biomarker in sweat (605b) uptaken by the sensor, and
a processing unit (607) configured to correct the determined value of biomarker concentration according to a correction model for estimating the biomarker concentration as released by the sweat gland based on at least one of the flow rate, the pH value and the rate of change of the pH value of the sweat to correct the biomarker concentration for reabsorption effects related to the flow rate and/or the pH value and/or the rate of change of the pH value, and/or the concentration of the analyte in the sweat.

13. Computer-implemented method of controlling a measurement mode of a sweat sensor, the method comprising the steps:
receiving medical data about the user by the sweat sensor (SI),
wherein the sweat sensor comprises a measurement unit configured for carrying out, based on the sweat uptaken by the sensor, at least one of a sweat rate measurement and a sweat rate proxy measurement,
the method further comprising the steps
generating a control signal based on the received medical data about the user (S2), and
causing, with the control signal, an adaptation of a measurement mode of the measurement unit of the sweat sensor for a next measurement cycle of the sweat sensor (S3).

14. Method according to claim 13,
wherein the measurement unit comprises at least two sensors, preferably all three sensors, of:
a) a flow sensor configured for carrying out the sweat rate measurement and for determining a sweat rate of the user,
b) a sweat pH sensor configured for determining the pH value of the uptaken sweat as a sweat rate proxy measurement, and
c) an analyte concentration sensor configured for determining a concentration of the analyte in the uptaken sweat, wherein the concentration of the analyte in the sweat is indicative for a sweat rate of the user,
and the method further comprising
selecting, for use in the next measurement cycle of the sweat sensor, either the flow sensor or the sweat pH sensor or the analyte concentration sensor based on the received medical data about the user (S4).

15. Use of medical data about a user of a sweat sensor in adapting, for a next measurement cycle of the sweat sensor, a measurement mode of a measurement unit of the sweat sensor, the measurement unit carrying out at least one of a sweat rate measurement and a sweat rate proxy measurement, which determines a sweat parameter of the user's sweat, which parameter is indicative for the sweat rate of the user.

16. Computer program element, which when being processed by a processor, causes the processor to carry out the following steps:
receiving medical data about a user of a sweat sensor by the sweat sensor comprising a measurement unit configured for carrying out, based on the sweat uptaken by the sensor, at least one of a sweat rate measurement and a sweat rate proxy measurement,
generating a control signal based on the received medical data about the user,
causing, with the control signal, an adaptation of a measurement mode of the measurement unit of the sweat sensor for a next measurement cycle of the sweat sensor.
